# EUROPEAN PATENT APPLICATION

(11) **EP 4 591 722 A1**
(43) Date of publication of application: **30.07.2025**
(21) Application number: 22959060.9
(22) Date of filing: 21.09.2022
(51) Int. Cl.: A23L 33/125, A23L 29/30

(54) **SACCHARIDE SUSTAINED-RELEASE COMPOSITION AND PREPARATION METHOD THEREFOR**

(71) Applicant: Zhejiang Hanmai Pharmaceutical Technology Co., Ltd., Shaoxing City, Zhejiang Province 312081 (CN)
(72) Inventor: ZHANG, Jing, Shanghai 200233 (CN)
(74) Representative: Currado, Luisa
(86) International application number: PCT/CN2022/120127
(87) International publication number: WO 2024/060048

(57) **Abstract**

A saccharide sustained-release composition and a preparation method therefor. Provided is the saccharide sustained-release composition. The composition comprises a saccharide substance and a sustained-release agent, the sustained-release agent comprising at least two selected from hydroxypropyl methylcellulose, sodium alginate, cellulose, methyl cellulose, ethyl cellulose, microcrystalline cellulose, carboxymethyl cellulose, sodium carboxymethyl cellulose, hydroxyethyl cellulose, hydroxypropyl cellulose, polyethylene oxide, a carboxyvinyl polymer, polyoxyethylene, a polyvinyl acetate aqueous dispersion, glycerol monooleate, glycerol monostearate, a carbomer homopolymer, a carbomer interpolymer, carrageenan, shellac, guar gum, xanthan gum, alginic acid and starch.

## Description

### TECHNICAL FIELD

The present disclosure relates to the field of preparation technology of sustained-release products, and in particular to a sustained-release saccharide composition and a method for preparing same.

### BACKGROUND

Diabetic hypoglycemia is a common complication of diabetes. It is reported that 24%-64% of patients with diabetes suffer from hypoglycemia of various severities that generally occurs at night. Furthermore, several clinical studies have shown that more than 50% of diabetic hypoglycemia events occur between 22:00 and 6:00 the next morning. Nocturnal hypoglycemia events are rarely intervened due to difficulties in observation and may thus cause high fasting glucose the next morning. In this case, a blind increase in the dose of hypoglycemics may result in serious consequences. In addition, elderly patients with diabetes need more attention, as the onset of hypoglycemia is often unnoticed due to the impaired ability of their central nervous system to recognize blood glucose levels, resulting in a prolonged duration of hypoglycemia and thus an increased incidence of fatal hypoglycemia.

Currently, a common method for preventing and controlling nocturnal hypoglycemia in patients with diabetes is to ingest high-carbohydrate foods such as biscuits, desserts, and the like before sleeping. However, by directly ingesting such foods, it may be difficult to control and maintain a stable nocturnal blood glucose concentration in different patients due to individual differences, specifically, differences in the digestive and absorption capacity for different types and amounts of carbohydrates in different patients; Furthermore, other substances may adversely affect the conditions of diabetes patients may exist in common foods, making common foods unsuitable for the patients. Therefore, in order to improve the universality and stability of interventions against diabetic hypoglycemia and avoid the adverse effects caused by taking common foods before sleeping, the development of a saccharide-containing food having stable sustained-release functionality is urgently needed.

In summary, there are two major technical problems in developing such a saccharide-containing food having sustained-release functionality:
The first one is to achieve the sustained release of saccharides in the human body. Saccharides such as glucose and maltose are soluble in water, and direct oral administration of such matters with high solubility may result in complete digestion and absorption in a short while. However, the occurrence of diabetic hypoglycemia is a slow and continuous process, thus requiring gradual and sustained-release effects of the products. In most studies, hydrophobic polymers were added in solid oral dosage forms to modulate the release of saccharides, which, however, can hardly be degraded in the human body and are often pH-dependent. The pH value changes in a stepwise manner in the gastrointestinal environment in the human body, which may be influenced by various factors in and outside the body, such as mental pressure, medicines influencing acid secretion, diseases, race, age, dietary habits, and so on. Therefore the pH dependence of such hydrophobic polymers added for modulating sustained saccharide release may result in individual differences in the sustained-release effect.
The second one is to ensure the stability of the product, i.e., the consistency in dissolution effects. Saccharides (such as maltose) are highly hygroscopic, and may easily absorb water even at a low relative humidity (e.g., a relative humidity of 40%), resulting in increased particle size and thus a change in dissolution properties; Furthermore, different saccharides may have different compressibility, and some saccharides (such as maltose) may have poor compressibility and cannot be easily tableted. Hence, it is necessary to select a suitable coating formulation and production process to compensate for the disadvantages of physical properties (e.g., high hygroscopicity and poor compressibility) of saccharides (such as glucose and maltose), so as to ensure the product stability.

Therefore, there is an urgent need in the art for a novel sustained-release saccharide composition having a suitable sustained-release effect, a high stability, a simple preparation process, etc.

### SUMMARY

The present disclosure provides a sustained-release saccharide composition, comprising a saccharide and a sustained-release agent, wherein the sustained-release agent comprises at least two selected from hydroxypropyl methylcellulose, sodium alginate, cellulose, methylcellulose, ethylcellulose, microcrystalline cellulose, carboxymethylcellulose, sodium carboxymethylcellulose, hydroxyethyl cellulose, hydroxypropyl cellulose, polyethylene oxide, carboxyvinyl polymer, polyoxyethylene, aqueous polyvinyl acetate dispersion, glycerol monooleate, glycerol monostearate, carbomer homopolymer, carbomer interpolymer, carrageenan, shellac, guar gum, xanthan gum, alginic acid, and starch.

In a preferred embodiment, the sustained-release saccharide composition further comprises a diluent and a glidant.

In another preferred embodiment, the sustained-release saccharide composition further comprises a diluent, a glidant, and a binder.

In a preferred embodiment, the saccharide in the sustained-release saccharide composition is selected from glucose, maltose, lactose, galactose, fructose, mannose, xylose, sucrose, fucose, arabinose, rhamnose, ribose, sorbose, lactulose, lactitol, tagatose, maltulose, palatinose, isomaltose, trehalose, sophorose, rutinose, kojibiose, nigerose, laminaribiose, gentiobiose, turanose, gentiobiulose, mannobiose, melibiulose, xylobiose, sorbitol, mannitol, and erythritol, and a combination thereof.

In a more preferred embodiment, the saccharide is present in a percentage of 45%-85%, preferably 60%-80%, by weight of the sustained-release composition.

In a most preferred embodiment, the saccharide is glucose or maltose, and is present in a percentage of 50%-80%, preferably 70%-80%, by weight of the sustained-release composition.

In a more preferred embodiment, the sustained-release agent is present in a percentage of 5%-40%, preferably 12%-24%, by weight of the sustained-release composition.

In a most preferred embodiment, the sustained-release agent is a combination of hydroxypropyl methylcellulose and sodium alginate, and is present in a percentage of 5%-35%, preferably 10%-30%, and more preferably 15%-25%, by weight of the sustained-release composition. Preferably, the viscosity of the hydroxypropyl methylcellulose is 75000-140000 mPa·s, preferably 90000-120000 mPa·s, and more preferably 100000 mPa·s; or the viscosity of the hydroxypropyl methylcellulose is 11250-21000 mPa·s, preferably 13000-18000 mPa·s, and more preferably 15000 mPa·s.

In a preferred embodiment, the sustained-release saccharide composition is in the form of a granule or a tablet; preferably, the granule or the tablet further comprises a coating part. More preferably, the coating part comprises a film-forming agent, a plasticizer, and an anti-adherent.

In a preferred embodiment, the film-forming agent in the coating part of the sustained-release saccharide composition is selected from hydroxypropyl methylcellulose, hydroxypropyl cellulose, sodium methylcellulose, an acrylate polymer, and a combination thereof.

In a more preferred embodiment, the film-forming agent is present in a percentage of 55%-95%, preferably 65%-85%, by weight of the coating part.

In a most preferred embodiment, the film-forming agent is hydroxypropyl methylcellulose, and is present in a percentage of 65%-95%, preferably 75%-85%, by weight of the coating part. Preferably, the viscosity of the hydroxypropyl methylcellulose is 1-20 mPa·s, preferably 2-15 mPa·s, more preferably 3-10 mPa·s, and most preferably 5 mPa·s.

In a preferred embodiment, the plasticizer in the coating part is selected from castor oil, coconut oil, glycerin, propylene glycol, low-molecular-weight polypropylene glycol, polysorbate, a sorbitan ester, triethyl citrate, diethyl phthalate, dibutyl phthalate, and tributyl citrate, and a combination thereof.

In a more preferred embodiment, the plasticizer is present in a percentage of 5%-20%, preferably 5%-15%, by weight of the coating part.

In a most preferred embodiment, the plasticizer is triethyl citrate, and is present in a percentage of 5%-18%, preferably 5%-10%, by weight of the coating part.

In a preferred embodiment, the anti-adherent in the coating part of the sustained-release saccharide composition is selected from magnesium stearate, magnesium silicate, stearic acid, glycerol stearate, glycerol monostearate, sodium stearate, talc, colloidal silica, magnesium oxide, magnesium trisilicate, and a combination thereof.

In a more preferred embodiment, the anti-adherent is present in a percentage of 2%-20%, preferably 5%-15%, by weight of the coating part.

In a most preferred embodiment, the anti-adherent is a combination of glycerol monostearate and magnesium stearate, and is present in a percentage of 2%-15%, preferably 2%-12%, by weight of the coating part.

In a preferred embodiment, the coating part comprises, by weight of the coating part: about 80.33% of hydroxypropyl methylcellulose, about 8.83% of triethyl citrate, about 2.81% of glycerol monostearate, and about 8.03% of magnesium stearate. Preferably, the viscosity of the hydroxypropyl methylcellulose is 5 mPa·s.

In a preferred embodiment, the sustained-release saccharide composition comprises a core part and a coating part. The core part comprises, by weight of the core part: about 79.24% of maltose, about 16.64% of hydroxypropyl methylcellulose, about 1.11% of sodium alginate, about 1.9% of microcrystalline cellulose, and about 1.11% of magnesium stearate. Preferably, the viscosity of the hydroxypropyl methylcellulose is 15000 mPa·s.

In another preferred embodiment, the sustained-release saccharide composition comprises a core part and a coating part. The core part comprises, by weight of the core part: about 71.43% of glucose, about 24% of hydroxypropyl methylcellulose, about 1% of sodium alginate, about 1.71% of microcrystalline cellulose, about 1% of magnesium stearate, and about 0.86% of hydroxypropyl methylcellulose. Preferably, the viscosity of the hydroxypropyl methylcellulose of about 24% is 100000 mPa·s, and the viscosity of the hydroxypropyl methylcellulose of about 0.86% is 15 mPa·s.

In a preferred embodiment, the sustained-release saccharide composition comprises a core part and a coating part, wherein the coating part is present in a percentage of 2-8%, preferably 3-6%, by weight of the core part.

In another preferred embodiment, the sustained-release saccharide composition comprises a core part and a coating part, wherein the coating part is present in a percentage of 15-25%, preferably 18-22%, by weight of the core part.

In a preferred embodiment, the releasing amount of the sustained-release saccharide composition is, on the basis of the original mass, less than 35%, preferably 25%, and more preferably 15% at 2 h post-dose; less than 50%, preferably 40%, and more preferably 35% at 4 h post-dose; greater than 50%, preferably 55%, and more preferably 60% at 8 h post-dose; and greater than 80%, preferably 90% at 24 h post-dose.

In a preferred embodiment, the dosage form of the sustained-release saccharide composition is a tablet, a pill, or a capsule, preferably a tablet.

In a preferred embodiment, the preparation for the core part of the sustained-release saccharide composition includes direct granulation, dry granulation, and wet granulation, wherein the wet granulation includes high-shear granulation, low-shear granulation, extrusion/spheronization granulation, melt granulation, and boiling granulation.

In a more preferred embodiment, the preparation method of the core part is direct granulation or high-shear granulation.

For different saccharides in the present disclosure, different optimal formulations that can achieve the optimal sustained-release effect are provided herein, i.e., a releasing amount of the sustained-release saccharide composition, on the basis of the original mass, preferably less than 35%, preferably 25%, and more preferably 15% at 2 h post-dose; preferably less than 50%, preferably 40%, and more preferably 35% at 4 h post-dose; preferably greater than 50%, preferably 55%, and more preferably 60% at 8 h post-dose; and preferably greater than 80%, preferably 90% at 24 h post-dose.

### BRIEF DESCRIPTION OF THE DRAWINGS

FIG. 1 illustrates sustained maltose release curves of the core part of sustained-release maltose compositions containing hydroxypropyl methylcellulose having different viscosities.
FIG. 2 illustrates sustained maltose release curves of coated and uncoated sustained-release maltose compositions.
FIG. 3 illustrates sustained maltose release curves of a sustained-release maltose composition in different pH conditions.
FIG. 4 illustrates sustained maltose release curves of a sustained-release maltose composition at different time points.
FIG. 5 illustrates sustained glucose release curves of sustained-release glucose compositions containing different sustained-release agents in different pH conditions.
FIG. 6 illustrates saccharide release curves of sustained-release compositions containing different saccharides.
FIG. 7 illustrates sustained glucose release curves of a sustained-release glucose composition with different coatings.
FIG. 8 illustrates sustained glucose release curves of a sustained-release glucose composition in different pH conditions.
FIG. 9 illustrates sustained glucose release curves of a sustained-release glucose composition at different time points.

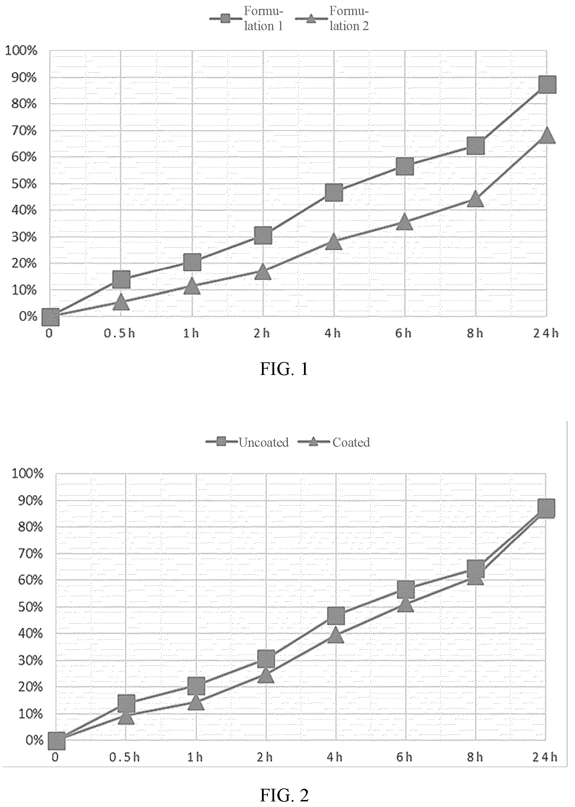

In FIGs. 1-9, the abscissa denotes the time for dissolution measurements, and the ordinate denotes the percentage of released drug by weight.

### DETAILED DESCRIPTION

In the present specification, unless otherwise specified, the involved components or preferred components thereof may be combined with each other to form new technical solutions.

In the present specification, unless otherwise specified, all the embodiments and preferred embodiments mentioned may be combined with each other to form new technical solutions.

In the present specification, unless otherwise specified, all the technical features and preferred features mentioned may be combined with each other to form new technical solutions.

In the present specification, unless otherwise specified, the term "a" or "an" refers to "at least one". In the present specification, unless otherwise specified, all tests mentioned are carried out at room temperature.

In the present specification, unless otherwise specified, all percentages, parts, and the like are on weight basis.

The "ranges" disclosed herein are delimited by lower and upper limits. There may be one or more lower limits and one or more upper limits. A given range is defined by selecting a lower limit and an upper limit. The selected lower and upper limits define the margins of a particular range. All ranges defined in this manner are inclusive and combinable. That is, any lower limit can be combined with any upper limit to form a range.

As used herein, a range defined by "about" is generally a range of experimental errors. For example, if the experimental error is 0.1, "about 7" means 7 ± 0.1.

Specifically, the present disclosure provides a sustained-release saccharide composition, comprising a saccharide and a sustained-release agent, wherein the sustained-release agent comprises at least two selected from hydroxypropyl methylcellulose, sodium alginate, cellulose, methylcellulose, ethylcellulose, microcrystalline cellulose, carboxymethylcellulose, sodium carboxymethylcellulose, hydroxyethyl cellulose, hydroxypropyl cellulose, polyethylene oxide, carboxyvinyl polymer, polyoxyethylene, aqueous polyvinyl acetate dispersion, glycerol monooleate, glycerol monostearate, carbomer homopolymer, carbomer interpolymer, carrageenan, shellac, guar gum, xanthan gum, alginic acid, and starch. The term "comprise" as used herein means that the medicine may also contain any other components, which can be present in any amount as long as the component present in the amount is acceptable in the human body and does not substantially affect the biological activity of the active ingredient in the pharmaceutical composition of the present disclosure.

**In** a preferred embodiment, the sustained-release saccharide composition further comprises a diluent and a glidant.

In another preferred embodiment, the sustained-release saccharide composition further comprises a diluent, a glidant, and a binder.

In a preferred embodiment, the saccharide in the sustained-release saccharide composition is selected from glucose, maltose, lactose, galactose, fructose, mannose, xylose, sucrose, fucose, arabinose, rhamnose, ribose, sorbose, lactulose, lactitol, tagatose, maltulose, palatinose, isomaltose, trehalose, sophorose, rutinose, kojibiose, nigerose, laminaribiose, gentiobiose, turanose, gentiobiulose, mannobiose, melibiulose, xylobiose, sorbitol, mannitol, and erythritol, and a combination thereof.

In a more preferred embodiment, the saccharide is present in a percentage of 45%-85%, preferably 60%-80%, by weight of the sustained-release composition.

In a most preferred embodiment, the saccharide is glucose or maltose, and is present in a percentage of 50%-80%, preferably 70%-80%, by weight of the sustained-release composition.

The term "sustained-release agent" refers to a substance in a formulation that delays the release of a particular substance (e.g., a saccharide as described herein) from the formulation. The sustained-release agent of the sustained-release saccharide composition disclosed herein is selected from at least two, such as two, three, or four, of hydroxypropyl methylcellulose, sodium alginate, cellulose, methylcellulose, ethylcellulose, microcrystalline cellulose, carboxymethylcellulose, sodium carboxymethylcellulose, hydroxyethyl cellulose, hydroxypropyl cellulose, polyethylene oxide, carboxyvinyl polymer, polyoxyethylene, aqueous polyvinyl acetate dispersion, glycerol monooleate, glycerol monostearate, carbomer homopolymer, carbomer interpolymer, carrageenan, shellac, guar gum, xanthan gum, alginic acid, and starch.

In a preferred embodiment, the sustained-release agent is present in a percentage of 5%-40%, preferably 12%-24%, by weight of the sustained-release composition.

In a more preferred embodiment, the sustained-release agent is a combination of hydroxypropyl methylcellulose and sodium alginate, and is present in a percentage of 5%-35%, preferably 10%-30%, and more preferably 15%-25%, by weight of the sustained-release composition. Preferably, the viscosity of the hydroxypropyl methylcellulose is 75000-140000 mPa·s, preferably 90000-120000 mPa·s, and more preferably 100000 mPa·s; or the viscosity of the hydroxypropyl methylcellulose is 11250-21000 mPa·s, preferably 13000-18000 mPa·s, and more preferably 15000 mPa·s.

In a preferred embodiment, the diluent is selected from microcrystalline cellulose.

The term "glidant" refers to a substance in a formulation that increases powder flow rate and reduces the agglomeration of powder. In the present application, the glidant of the sustained-release saccharide composition is selected from magnesium stearate, stearic acid, calcium stearate, sodium stearate, zinc stearate, glycerol stearate, glycerol distearate, glycerol tristearate, myristic acid, palmitic acid, polyethylene glycol, polysorbate 20, polysorbate 40, polysorbate 60, polysorbate 80, polyoxyethylene 10 oleyl ether, polyoxyethylene 15 hydroxystearate, polyoxyethylene 20 cetyl ether, polyoxyethylene 40 stearate, potassium benzoate, sodium benzoate, sorbitan monolaurate, sorbitan monooleate, sorbitan monopalmitate, sorbitan monostearate, sorbitan sesquioleate, sorbitan trioleate, talc, colloidal silica, tribasic calcium phosphate, calcium silicate, cellulose, magnesium silicate, magnesium oxide, silica, magnesium trisilicate, sodium dodecyl sulfate, sodium stearyl fumarate, magnesium dodecyl sulfate, and a combination thereof.

In a preferred embodiment, the glidant in the sustained-release saccharide composition is selected from magnesium stearate.

The term "binder" refers to a solid powder or viscous liquid that binds non-viscous or less viscous materials into granules or for compression molding. In the present application, the binder in the sustained-release saccharide composition is selected from hydroxypropyl methylcellulose, ethylcellulose, hydroxypropyl cellulose, vinyl cellulose, hydroxyethyl methylcellulose, polyethylene glycol, hydroxyethyl cellulose, calcium carboxymethylcellulose, calcium cellulose glycolate, carmellose calcium, chitosan hydrochloride, sodium carboxymethylcellulose, magnesium aluminum silicate, methylcellulose, polyoxyethylene, sodium alginate, starch, corn starch, pregelatinized starch, hydroxypropyl starch, dextrin, maltodextrin, glucose, sucrose, compressible sugar, zein, gelatin, guar bean gum, guar gum, polymethacrylate, sorbitol, gum arabic, carbomer, polyvinylpyrrolidone, copovidone, povidone, keratin powder, inulin, and a combination thereof.

In a preferred embodiment, the binder in the sustained-release saccharide composition is hydroxypropyl methylcellulose. Preferably, the viscosity of the hydroxypropyl methylcellulose is 1-100 mPa·s, preferably 5-50 mPa·s, more preferably 10-30 mPa·s, and most preferably 12-20 mPa·s, for example, 15 mPa·s.

**In** a preferred embodiment, the sustained-release saccharide composition is in the form of a granule or a tablet; preferably, the granule or the tablet further comprises a coating part. More preferably, the coating part comprises a film-forming agent, a plasticizer, and an anti-adherent. As used herein, the percentages of the saccharide, sustained-release agent, diluent, glidant, and binder are generally calculated by weight of the sustained-release saccharide composition without the coating part.

The term "film-forming agent" refers to a polymer or material capable of forming a continuous film. **In** the present application, the film-forming agent in the coating part is selected from hydroxypropyl methylcellulose, hydroxypropyl cellulose, sodium methylcellulose, an acrylate polymer, and a combination thereof.

**In** a preferred embodiment, the film-forming agent is present in a percentage of 55%-95%, preferably 65%-85%, by weight of the coating part.

**In** a more preferred embodiment, the film-forming agent is hydroxypropyl methylcellulose, and is present in a percentage of 65%-95%, preferably 75%-85%, by weight of the coating part. Preferably, the viscosity of the hydroxypropyl methylcellulose is 1-20 mPa·s, preferably 2-15 mPa·s, more preferably 3-10 mPa·s, and most preferably 5 mPa·s.

The term "plasticizer" refers to a substance in a formulation that alters the flexibility, tensile strength, or adhesion of the film formed. **In** the present application, the plasticizer in the coating part of the sustained-release saccharide composition is selected from castor oil, coconut oil, glycerin, propylene glycol, low-molecular-weight polypropylene glycol, polysorbate, a sorbitan ester, triethyl citrate, diethyl phthalate, dibutyl phthalate, tributyl citrate, and a combination thereof.

In a preferred embodiment, the plasticizer is present in a percentage of 5%-20%, preferably 5%-15%, by weight of the coating part.

In a more preferred embodiment, the plasticizer is triethyl citrate, and is present in a percentage of 5%-18%, preferably 5%-10%, by weight of the coating part.

The term "anti-adherent" refers to a substance that reduces the adhesion of a solid dosage form during the coating. In the present application, the anti-adherent in the coating part is selected from magnesium stearate, magnesium silicate, stearic acid, glycerol stearate, glycerol monostearate, sodium stearate, talc, colloidal silica, magnesium oxide, magnesium trisilicate, and a combination thereof.

In a more preferred embodiment, the anti-adherent is present in a percentage of 2%-20%, preferably 5%-15%, by weight of the coating part.

In a most preferred embodiment, the anti-adherent is a combination of glycerol monostearate and magnesium stearate, and is present in a percentage of 2%-15%, preferably 2%-12%, by weight of the coating part.

The term "diluent" refers to an ingredient in a formulation for increasing the volume or the weight. In the present application, the diluent in the coating part is selected from microcrystalline cellulose, ethylcellulose, powdered cellulose, cellulose acetate, silicified microcrystalline cellulose, cellulose calcium salt, anhydrous lactose, lactose monohydrate, glucose, polydextrose, sucrose, isomaltose, compressible sugar, fructose, trehalose, corn syrup, lactitol, xylitol, erythritol, sorbitol, starch, pregelatinized starch, corn starch, wheat starch, starch hydrolysate, dextrin, maltodextrin, anhydrous calcium phosphate, calcium carbonate, calcium sulfate, calcium lactate, calcium phosphate dihydrate, tribasic calcium phosphate, ethyl acrylate, α-lactalbumin, magnesium carbonate, magnesium oxide, sodium chloride, dimethicone, methacrylic acid, ethyl acrylate copolymer, methyl methacrylate copolymer, amino methacrylate copolymer, methyl methacrylate copolymer dispersion, and a combination thereof.

In a preferred embodiment, the sustained-release saccharide composition comprises a core part and a coating part. The coating part comprises, by weight of the coating part: about 80.33% of hydroxypropyl methylcellulose, about 8.83% of triethyl citrate, about 2.81% of glycerol monostearate, and about 8.03% of magnesium stearate, wherein the viscosity of the hydroxypropyl methylcellulose is 5 mPa·s.

In a preferred embodiment, the sustained-release saccharide composition comprises a core part and a coating part. The core part comprises, by weight of the core part: about 79.24% of maltose, about 16.64% of hydroxypropyl methylcellulose, about 1.11% of sodium alginate, about 1.9% of microcrystalline cellulose, and about 1.11% of magnesium stearate, wherein the viscosity of the hydroxypropyl methylcellulose is 15000 mPa·s.

In another preferred embodiment, the sustained-release saccharide composition comprises a core part and a coating part. The core part comprises, by weight of the core part: about 71.43% of glucose, about 24% of hydroxypropyl methylcellulose, about 1% of sodium alginate, about 1.71% of microcrystalline cellulose, about 1% of magnesium stearate, and about 0.86% of hydroxypropyl methylcellulose, wherein, the viscosity of the hydroxypropyl methylcellulose of about 24% is 100000 mPa·s, and the viscosity of the hydroxypropyl methylcellulose of about 0.86% is 5 mPa·s.

In a preferred embodiment, the sustained-release saccharide composition comprises a core part and a coating part. The coating part of the sustained-release saccharide composition is present in a percentage of 2%-8%, preferably 3%-6%, by weight of the core part.

In another preferred embodiment, the sustained-release saccharide composition comprises a core part and a coating part. The coating part of the sustained-release saccharide composition is present in a percentage of 15%-25%, preferably 18%-22%, by weight of the core part. In general, the release of a medicine in a tablet dosage form follows the first-order release kinetic model. That is, initially, the release of the medicine is quick before 60%-80% of the medicine is released, and subsequently, the release of the medicine is slowed. Therefore, in an experiment for detecting the dissolution, more detection time points will be arranged at the early stage with shorter time intervals, and the time intervals of the detection are prolonged after most of the medicine is released. For the human body, the medicine absorption may require up to 6-8 h. Therefore, in the present application, 0 h, 0.5 h, 1 h, 2 h, 4 h, 6 h, 8 h, and 24 h were set as the time points for detecting the dissolution, so as to perceive the release of the medicine in the sustained-release saccharide composition.

The sustained-release saccharide composition of the present disclosure features a stable sustained-release profile, which matches with the characteristic nocturnal hypoglycemia in diabetes patients, such that the use of the composition before sleep can effectively control the blood glucose, so as to prevent and control nocturnal hypoglycemia.

In a preferred embodiment, the releasing amount of the sustained-release saccharide composition is, on the basis of the original mass, less than 35%, preferably 25%, and more preferably 15% at 2 h post-dose; less than 50%, preferably 40%, and more preferably 35% at 4 h post-dose; greater than 50%, preferably 55%, and more preferably 60% at 8 h post-dose; and greater than 80%, preferably 90% at 24 h post-dose.

In a preferred embodiment, the dosage form of the sustained-release saccharide composition is a tablet, a pill, or a capsule, preferably a tablet.

In a preferred embodiment, the preparation for the core part of the sustained-release saccharide composition includes direct granulation, dry granulation, and wet granulation, wherein the wet granulation includes high-shear granulation, low-shear granulation, extrusion/spheronization granulation, melt granulation, and boiling granulation.

In a more preferred embodiment, the preparation method of the core part is direct granulation or high-shear granulation. The direct granulation avoids two factors temperature and moisture that are unfavorable for the stability of medicines, and reduces the time consumption of the process and the requirements of equipment and space.

The present disclosure will be further illustrated in detail with reference to the following examples. However, it will be appreciated that these examples are provided for illustration only and are not intended to limit the scope of the present disclosure.

In the examples, the materials used are shown in Table 1 below:

**Table 1: Materials**

| Material | Trade name | Manufacturer |
|---|---|---|
| Maltose | Maltose | Baolingbao Biology |
| Hydroxypropyl methylcellulose (viscosity: 15000 mPa·s) | Hydroxypropyl methylcellulose (75RT15000) | Rutocel, Tai'an |
| Hydroxypropyl methylcellulose (viscosity: 100000 mPa·s) | HPMC K100M | Rutocel, Tai'an |
| Sodium alginate | Sodium alginate | Hyzlin, Qingdao |
| Microcrystalline cellulose | Microcrystalline cellulose | Xinda Biotech, Shandong |
| Magnesium stearate | Magnesium stearate | Xinda Biotech, Shandong |
| Hydroxypropyl methylcellulose (viscosity: 5 mPa·s) | HPMC 5cps | Rutocel, Tai'an |
| Triethyl citrate | Triethyl citrate | Kangqin Biotech, Shandong |
| Glycerol monostearate | Glycerol monostearate | Zhongnan, Anhui |
| Ethanol | Ethanol | Taicang Xintai |
| Glucose | Glucose | Fengtai, Shandong |
| Carbomer | Carbomer | Xi'an Tianzheng Medicinal Materials |
| Hydroxypropyl methylcellulose (viscosity: 15 mPa·s) | HPMC 15 cps | Rutocel, Tai'an |
| Maltitol | Maltitol | Lüjian Biotech, Shandong |
| Ethylcellulose | Ethylcellulose | Colorcon |

### Example 1: Formulation of sustained-release maltose composition

Tablets were prepared according to the formulations of the sustained-release maltose compositions shown in Table 2 below. In the formulations, the sustained-release agent plays an important role in controlling the regular and slow release of the medicine. Therefore, the dissolution profile of maltose in the sustained-release compositions was investigated with combinations of sodium alginate with two hydroxypropyl methylcellulose products having different viscosities (viscosity: 15000 mPa·s and 100000 mPa·s) used as the sustained-release agent (the formulation is shown in Table 2).

**Table 2: Formulations of sustained-release maltose compositions containing hydroxypropyl methylcellulose having different viscosities**

| **Component** | **Material** | **Formulation 1** | | **Formulation 2** | |
|---|---|---|---|---|---|
| | | **Amount (mg)** | **Proportion (%)** | **Amount (mg)** | **Proportion (%)** |
| Saccharide | Maltose | 1000.0 | 79.24 | 1000.0 | 79.24 |
| Sustained-release agent | Hydroxypropyl methylcellulose (viscosity: 15000 mPa·s) | 210.0 | 16.64 | - | - |
| | Hydroxypropyl methylcellulose (viscosity: 100000 mPa·s) | - | - | 210.0 | 16.64 |
| | Sodium alginate | 14.0 | 1.11 | 14.0 | 1.11 |
| Diluent | Microcrystalline cellulose | 24.0 | 1.90 | 24.0 | 1.90 |
| Glidant | Magnesium stearate | 14.0 | 1.11 | 14.0 | 1.11 |

| | | | | | |
|---|---|---|---|---|---|
| Note: all the materials were of food grade. | | | | | |

The tablets of the two formulations were tested for dissolution according to "method 1 (the basket method)" recorded in the General Chapter 0931 - Dissolution and Drug Release Test in Chinese Pharmacopoeia (2020 Edition, Volume IV) using the peroxidase-glucose oxidase (PGO) technique. The specific conditions are as follows:
(1) Method 1 (basket method): The test was conducted in 500 mL of dissolution medium with a basket speed of 50 rpm and a solution temperature of 37.5 °C. The dissolution medium was a HCl solution at pH 1.2 for the first 2 h and a potassium dihydrogen phosphate-sodium hydroxide buffer at pH 6.8 for the last 22 h.
(2) Peroxidase-glucose oxidase (PGO) method: maltose was converted to glucose under the treatment of α-glucosidase; a PGO reagent was added, and the resulting mixture, together with the glucose standard containing the PGO reagent, was incubated for 10 min at 37 °C for color development, and the absorbance of the two at 520 nm was obtained by scanning.

The sample concentration was obtained according to the measured absorbance, the percentage of the dissolved maltose by weight was calculated (see Table 3), and the releasing profile was plotted (see FIG. 1).

**Table 3: Maltose dissolution results for sustained-release maltose compositions containing hydroxypropyl methylcellulose having different viscosities**

| Formulation | Percentage of dissolved maltose by weight (%) | | | | | | | |
|---|---|---|---|---|---|---|---|---|
| | 0 | 0.5h | 1h | 2h | 4h | 6h | 8h | 24h |
| 1 | 0 | 13.8 | 20.6 | 30.5 | 46.8 | 56.7 | 64.2 | 87.2 |
| 2 | 0 | 5.5 | 11.5 | 16.9 | 28.3 | 35.6 | 44.3 | 68.3 |

| | | | | | | | | |
|---|---|---|---|---|---|---|---|---|
| Note: the test conditions were HCl, pH 1.2 (2 h) + phosphate buffer, pH 6.8 (22 h). | | | | | | | | |

As can be seen from Table 3 and FIG. 1, both formulations 1 and 2 achieved the sustained-release of the saccharide. Compared with formulation 2, the composition of formulation 1 exhibited a slow release in 8 h but a greater overall dissolution amount within 24 h, specifically, on the basis of the original mass, a 2-h releasing amount less than 35%, a 4-h releasing amount less than 50%, an 8-h releasing amount greater than 60%, and a 24-h releasing amount greater than 80%, which are more desirable for the characteristic nocturnal hypoglycemia of the diabetes.

### Example 2: Coated tablet of sustained-release maltose composition

A coating was added to the sustained-release maltose composition tablet of formulation 1 of Example 1 according to the formulation of Table 4 below. The coating accounted for 4% ± 1% by weight of the sustained-release maltose composition tablet (excluding the coating part). In order to further investigate whether the coating has an influence on the release properties of the sustained-release maltose composition, the dissolution characteristics of maltose in coated or uncoated sustained-release composition were investigated.

**Table 4: Formulation of coating part of sustained-release maltose composition**

| Component | Material | Amount (mg) | Proportion (%) |
|---|---|---|---|
| Film-forming agent | Hydroxypropyl methylcellulose (viscosity: 5 mPa·s) | 40.9 | 80.33 |
| Plasticizer | Triethyl citrate | 4.5 | 8.83 |
| Anti-adherent | Glycerol monostearate | 1.4 | 2.81 |
| | Magnesium stearate | 4.1 | 8.03 |
| Solvent | Ethanol | Sufficient | / |
| | Water | Sufficient | / |

| | | | |
|---|---|---|---|
| Note: all the materials were of food grade. | | | |

The sustained-release maltose compositions prepared according to the two coating formulations were tested for dissolution according to "method 1 (the basket method)" recorded in the General Chapter 0931 - Dissolution and Drug Release Test in Chinese Pharmacopoeia (2020 Edition, Volume IV) using the peroxidase-glucose oxidase (PGO) technique. The specific conditions are as described in Example 1.

By the above method, the percentage of dissolved maltose by weight was calculated (Table 5) and the time curve was plotted (FIG. 2).

**Table 5: Maltose dissolution results for coated and uncoated sustained-release maltose compositions**

| Component | Percentage of dissolved maltose by weight (%) | | | | | | | |
|---|---|---|---|---|---|---|---|---|
| | 0 | 0.5h | 1h | 2h | 4h | 6h | 8h | 24h |
| Uncoated | 0 | 13.8 | 20.6 | 30.5 | 46.8 | 56.7 | 64.2 | 87.2 |
| Coated | 0 | 9.2 | 14.4 | 24.7 | 39.5 | 51.2 | 61.3 | 86.4 |

| | | | | | | | | |
|---|---|---|---|---|---|---|---|---|
| Note: the test conditions were HCl, pH 1.2 (2 h) + phosphate buffer, pH 6.8 (22 h). | | | | | | | | |

As can be seen from Table 5 and FIG. 2, the coated and uncoated tablets exhibited similar maltose dissolution, the releasing profile of maltose in the coated sustained-release maltose composition was similar to that in the uncoated tablet, specifically, on the basis of the original mass, a 2-h releasing amount less than 35%, a 4-h releasing amount less than 50%, an 8-h releasing amount greater than 60%, and a 24-h releasing amount greater than 80%, which are more desirable for the characteristic nocturnal hypoglycemia of the diabetes.

### Example 3: Preparation of sustained-release maltose composition

The amounts of materials of the tablet core part are shown in formulation 1 in Example 1, and the formulation of the coating part is shown in Table 4 in Example 2. The preparation procedures are detailed as follows:
(1) Core part
   1) Blending: Proper amounts of maltose, microcrystalline cellulose, hydroxypropyl methylcellulose (viscosity: 15000 mPa·s), and sodium alginate according to the formulation were sieved through a screen mesh; after sieving, the materials were mixed in a blender for 5 minutes; finally, a proper amount of magnesium stearate according to the formulation was sieved and added into the blender for lubrication, and the mixture was blended for 5 min.
   2) Tableting: The compositions were tableted in a high-speed tablet press using a standard female punch (20 mm × 10 mm), with the average tablet weight controlled at about 1200 mg and the hardness controlled at 80-280 N.
(2) Coating part
   a. Preparation of coating solution: Proper amounts of hydroxypropyl methylcellulose (viscosity: 5 mPa·s) and triethyl citrate according to the formulation were added into an ethanol solution before water was slowly added, and the mixture was uniformly mixed; proper amounts of magnesium stearate and glycerol monostearate according to the formulation were added into another ethanol solution, and the mixture was homogenized; finally, the two solutions were mixed to give the coating solution.
   b. Coating: tablet cores were loaded onto a coating machine and pre-heated at 40 ± 1 °C; then the coating solution was sprayed on the pre-heated tablet cores to give a uniform coating until a weight gain of 3%-5%; finally, the coated tablets were dried at 40 ± 1 °C for 120 min.

### Example 4: Dissolution property exploration of sustained-release maltose composition

The sustained-release maltose composition was tested for dissolution in media at three different pH gradients simulating the pH of 3 gastric (acidic) and intestinal (basic) circumstances.

The sustained-release maltose composition was tested at 3 different pH conditions for dissolution according to "method 1 (the basket method)" recorded in the General Chapter 0931 - Dissolution and Drug Release Test in Chinese Pharmacopoeia (2020 Edition, Volume IV) using the peroxidase-glucose oxidase (PGO) technique. The specific test conditions are detailed in Example 1. For condition 1 in Table 6, the dissolution medium was a HCl solution at pH 1.2 for the first 2 h and a sodium acetate trihydrate-glacial acetic acid buffer at pH 5.5 for the last 22 h; for condition 2 in Table 6, the dissolution medium was a HCl solution at pH 1.2 for the first 2 h and a potassium dihydrogen phosphate-sodium hydroxide buffer at pH 6.8 for the last 22 h; for condition 3 in Table 6, the dissolution medium was a HCl solution at pH 1.2 for the first 2 h and a potassium dihydrogen phosphate-sodium hydroxide buffer at pH 7.4 for the last 22 h.

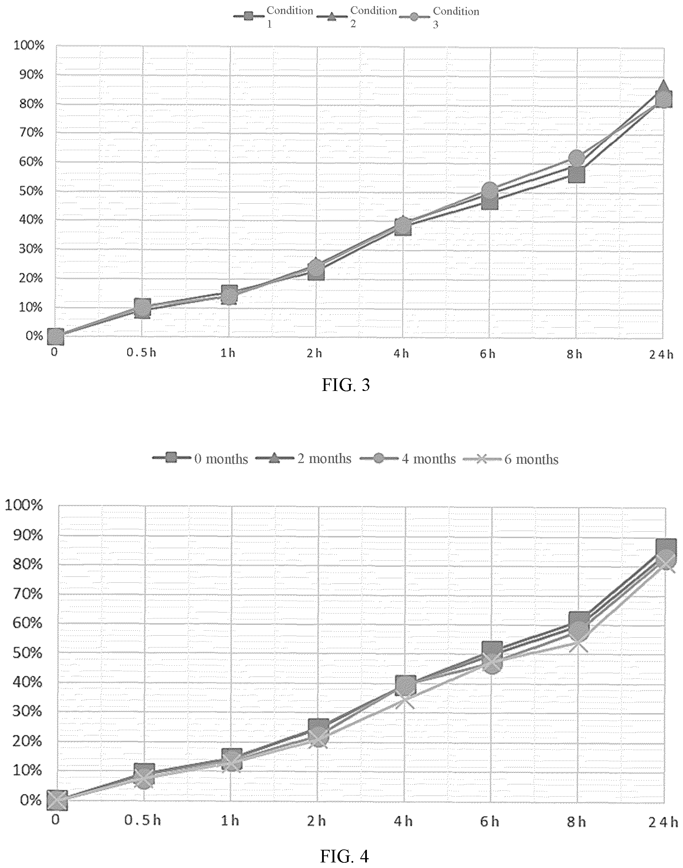

The dissolution amount of the sustained-release maltose composition over 24 hours was measured using the method described above (Table 6), and the maltose releasing profiles were plotted (as shown in FIG. 3).

**Table 6: Maltose dissolution results for sustained-release maltose composition in different pH conditions**

| Time (h) | Releasing proportion (%) | | |
|---|---|---|---|
| | Condition 1 | Condition 2 | Condition 3 |
| | HCl, pH 1.2 (2 h) + acetic acid buffer, pH 5.5 (22 h) | HCl, pH 1.2 (2 h) + phosphate buffer, pH 6.8 (22 h) | HCl, pH 1.2 (2 h) + phosphate buffer, pH 7.4 (22 h) |
| 0 | 0.0 | 0.0 | 0.0 |
| 0.5 | 10.2 | 9.2 | 10.3 |
| 1 | 15.3 | 14.4 | 13.8 |
| 2 | 22.8 | 24.7 | 24.3 |
| 4 | 38.2 | 39.5 | 39.0 |
| 6 | 47.1 | 51.2 | 51.1 |
| 8 | 56.6 | 61.3 | 62.2 |
| 24 | 82.7 | 86.4 | 82.3 |

As can be seen from Table 6 and FIG. 3, in all pH conditions, the sustained-release maltose composition exhibited, on the basis of the original mass, a 2-h releasing amount less than 25%, a 4-h releasing amount less than 40%, an 8-h releasing amount greater than 55%, and a 24-h releasing amount greater than 80%. The above results suggest that on one hand, the sustained-release maltose composition is not susceptible to changes in pH condition and is adaptive to the gastrointestinal tract environment with acidity changes; on the other hand, the releasing profile of maltose in the sustained-release maltose composition matches with the characteristic nocturnal hypoglycemia in diabetes patients, such that the use of the composition before sleep can effectively control the blood glucose, so as to prevent and control nocturnal hypoglycemia.

### Example 5: Stability test of sustained-release maltose composition

The stability of the sustained-release maltose composition prepared in Example 3 was investigated by dissolution test and weight gain test.

The prepared sustained-release maltose composition was tested for dissolution according to "method 1 (the basket method)" recorded in the General Chapter 0931 - Dissolution and Drug Release Test in Chinese Pharmacopoeia (2020 Edition, Volume IV) using the peroxidase-glucose oxidase (PGO) technique. The specific conditions are detailed in Example 1. The releasing data of maltose from the sustained-release maltose composition at baseline and after two, four, and six months (as shown in Table 7) were obtained, and the maltose releasing profiles were plotted (as shown in FIG. 4).

**Table 7: Maltose dissolution results for sustained-release maltose composition at different time**

| Time | Percentage of dissolved maltose by weight (%) | | | | | | | |
|---|---|---|---|---|---|---|---|---|
| | 0 | 0.5h | 1h | 2h | 4h | 6h | 8h | 24h |
| 0 | 0 | 9.2 | 14.4 | 24.7 | 39.5 | 51.2 | 61.3 | 86.4 |
| 2 months | 0 | 9.0 | 14.0 | 24.9 | 39.5 | 49.7 | 59.6 | 84.1 |
| 4 months | 0 | 7.5 | 13.6 | 22.0 | 39.6 | 47.0 | 58.0 | 82.8 |
| 6 months | 0 | 7.6 | 12.7 | 21.0 | 34.5 | 47.4 | 54.1 | 80.9 |

| | | | | | | | | |
|---|---|---|---|---|---|---|---|---|
| Note: the test conditions were HCl, pH 1.2 (2 h) + phosphate buffer, pH 6.8 (22 h). | | | | | | | | |

As can be seen from Table 7 and FIG. 4, after six months of preservation, the dissolution of maltose was substantially identical to that at baseline, i.e., on the basis of the original mass, a 2-h releasing amount less than 25%, a 4-h releasing amount less than 40%, an 8-h releasing amount greater than 50%, and a 24-h releasing amount greater than 80%.

Moreover, a long-term test (temperature: 25 °C; humidity: 60%) and an accelerated test (temperature: 40 °C; humidity: 75%) were conducted to determine changes in the appearance of the sustained-release maltose composition, so as to determine the stability of the sustained-release maltose composition (as shown in Table 8).

**Table 8: Changes in appearance of sustained-release maltose composition**

| Test condition | Coated or uncoated | Deliquesce or not | | | | |
|---|---|---|---|---|---|---|
| | | D0 | D1 | D2 | D7 | D14 |
| Temperature: 25 °C | Uncoated | No | No | No | No | No |
| Humidity: 60% | Coated | No | No | No | No | No |
| Temperature: 40 °C | Uncoated | No | No | Yes | Yes | Yes |
| Humidity: 75% | Coated | No | No | No | No | Yes |

As can be seen from Table 8, in the long-term test conditions, the sustained-release composition did not deliquesce within 14 days regardless of the presence or absence of the coating. However, in the accelerated test, the uncoated sustained-release composition started to deliquesce on day 2, while deliquescence was first observed in the coated sustained-release composition on day 14. Thus, the coating is effective in improving the stability of the sustained-release maltose composition.

### Example 6: Formulation of sustained-release glucose composition

Tablets of the sustained-release glucose compositions shown in Table 9 below were prepared according to the method in Example 1.

**Table 9: Formulation of sustained-release glucose composition containing different sustained-release agents**

| **Component** | **Material** | **Formulation 1** | | **Formulation 2** | |
|---|---|---|---|---|---|
| | | **Amount (mg)** | **Proportion (%)** | **Amount (mg)** | **Proportion (%)** |
| Saccharide | Glucose | 1000.0 | 71.43 | 1000.0 | 71.43 |
| Sustained-release agent | Carbomer | 350.0 | 25.00 | - | - |
| | Hydroxypropyl methylcellulose (viscosity: 100000 mPa·s) | - | - | 336.0 | 24.00 |
| | Sodium alginate | - | - | 14.0 | 1.00 |
| Diluent | Microcrystalline cellulose | 24.0 | 1.71 | 24.0 | 1.71 |
| Glidant | Magnesium stearate | 14.0 | 1.00 | 14.0 | 1.00 |
| Binder | Hydroxypropyl methylcellulose (viscosity: 15 mPa·s) | 12.0 | 0.86 | 12.0 | 0.86 |

| | | | | | |
|---|---|---|---|---|---|
| Note: all the materials were of pharmaceutical grade. | | | | | |

The tablets of the 2 formulations were tested for dissolution according to "method 1 (the basket method)" recorded in the General Chapter 0931 - Dissolution and Drug Release Test in Chinese Pharmacopoeia (2020 Edition, Volume IV) using the peroxidase-glucose oxidase (PGO) technique. The specific conditions are as follows:
(1) Method 1 (basket method): The test was conducted in 500 mL of dissolution medium with a basket speed of 50 rpm and a solution temperature of 37.5 °C. In condition 1 and condition 3 in Table 9, the dissolution medium was water at pH 6.5; in condition 2 and condition 4 in Table 9, the dissolution medium was a HCl solution at pH 1.2 for the first 2 h and a potassium dihydrogen phosphate-sodium hydroxide buffer at pH 6.8 for the last 22 h.
(2) Peroxidase-glucose oxidase (PGO) method: a PGO reagent was added to the sample processed in (1), and the resulting mixture, together with the glucose standard containing the PGO reagent, was incubated for 10 min at 37 °C for color development, and the absorbance of the two at 520 nm was obtained by scanning.

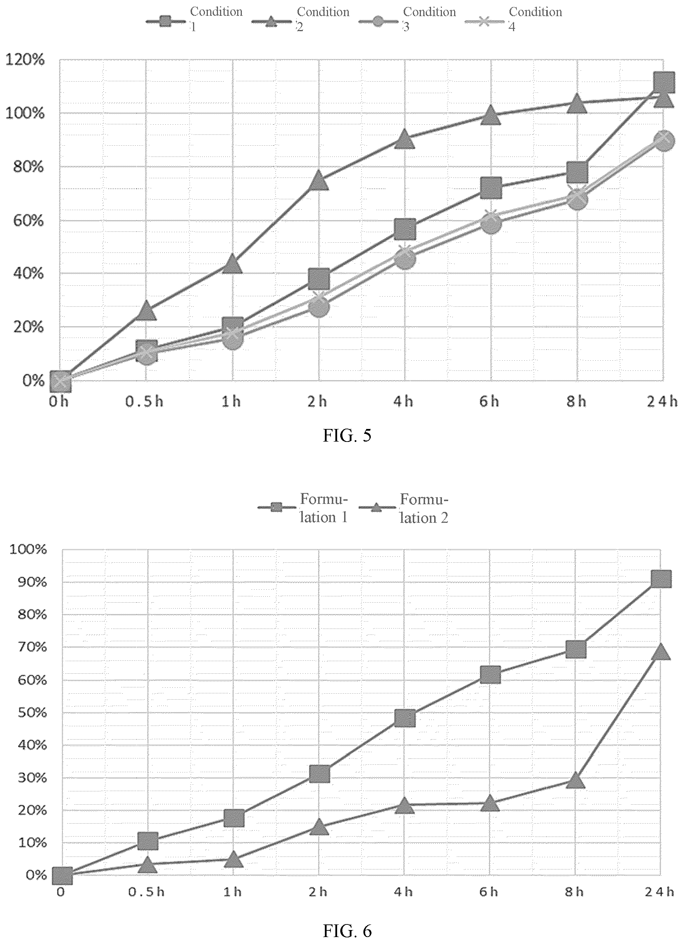

The sample concentration was obtained according to the measured absorbance, the percentage of the dissolved glucose by weight was calculated (see Table 10), and the releasing profile was plotted (see FIG. 5).

**Table 10: Glucose dissolution results for sustained-release glucose composition containing different sustained-release agents in different pH conditions**

| Time (h) | Releasing proportion (%) | | | |
|---|---|---|---|---|
| | Condition 1: Formulation 1 Water, pH 6.5 (24 h) | Condition 2: Formulation 1 HCl, pH 1.2 (2 h) + phosphate buffer, pH 6.8 (22 h) | Condition 3: Formulation 2 Water, pH 6.5 (24 h) | Condition 4: Formulation 2 HCl, pH 1.2 (2 h) + phosphate buffer, pH 6.8 (22 h) |
| 0 | 0 | 0 | 0 | 0 |
| 0.5 | 11.3 | 26.3 | 9.9 | 10.6 |
| 1 | 20.0 | 43.9 | 15.6 | 17.8 |
| 2 | 38.3 | 74.9 | 27.6 | 31.2 |
| 4 | 56.8 | 90.6 | 45.8 | 48.4 |
| 6 | 72.2 | 99.3 | 58.9 | 61.7 |
| 8 | 78.0 | 103.9 | 67.9 | 69.4 |
| 24 | 111.7 | 105.9 | 89.90 | 91.10 |

As can be seen from Table 10 and FIG. 5, the prepared glucose tablet of formulation 1 exhibited a great difference in glucose dissolution in different pH conditions; the glucose tablet of formulation 2 exhibited similar glucose dissolutions in all pH conditions, i.e., on the basis of the original mass, a 2-h releasing amount less than 35%, a 4-h releasing amount less than 50%, an 8-h releasing amount greater than 60%, and a 24-h releasing amount greater than 80%.

### Example 7: Comparison of sustained-release composition formulations of different saccharides

The glucose of formulation 2 in Example 6 was replaced with maltitol, and the dissolution profiles of the saccharides in the two sustained-release compositions were investigated. The two tablet formulations are shown in Table 11 below.

**Table 11: Sustained-release composition formulations of different saccharides**

| **Component** | **Material** | **Formulation 1** | | **Formulation 2** | |
|---|---|---|---|---|---|
| | | **Amount (mg)** | **Proportion (%)** | **Amount (mg)** | **Proportion (%)** |
| Saccharide | Glucose | 1000.0 | 71.43 | - | - |
| | Maltitol | - | - | 1000.0 | 71.43 |
| Sustained-release agent | Hydroxypropyl methylcellulose (viscosity: 100000 mPa·s) | 336.0 | 24.00 | 336.0 | 24.00 |
| | Sodium alginate | 14.0 | 1.00 | 14.0 | 1.00 |
| Diluent | Microcrystalline cellulose | 24.0 | 1.71 | 24.0 | 1.71 |
| Glidant | Magnesium stearate | 14.0 | 1.00 | 14.0 | 1.00 |
| Binder | Hydroxypropyl methylcellulose (viscosity: 15 mPa·s) | 12.0 | 0.86 | 12.0 | 0.86 |

| | | | | | |
|---|---|---|---|---|---|
| Note: all the materials were of food grade. | | | | | |

The sustained-release composition of the 2 formulations was tested for dissolution according to "method 1 (the basket method)" recorded in the General Chapter 0931 - Dissolution and Drug Release Test in Chinese Pharmacopoeia (2020 Edition, Volume IV) using the peroxidase-glucose oxidase (PGO) technique. The specific test conditions for glucose are detailed in Example 6, and the specific test conditions for maltitol are detailed in Example 1. The dissolution medium was a HCl solution at pH 1.2 for the first 2 h and a potassium dihydrogen phosphate-sodium hydroxide buffer at pH 6.8 for the last 22 h.

By the above method, the percentage of dissolved saccharides by weight was calculated (Table 12) and the time curve was plotted (FIG. 6).

**Table 12: Saccharide dissolution results for sustained-release compositions containing different saccharides**

| Formulation | Percentage of dissolved saccharide by weight (%) | | | | | | | |
|---|---|---|---|---|---|---|---|---|
| | 0 | 0.5h | 1h | 2h | 4h | 6h | 8h | 24h |
| Formulation 1 | 0 | 10.6 | 17.8 | 31.2 | 48.4 | 61.7 | 69.4 | 91.1 |
| Formulation 2 | 0 | 3.3 | 4.9 | 15.0 | 21.7 | 22.2 | 29.3 | 68.8 |

| | | | | | | | | |
|---|---|---|---|---|---|---|---|---|
| Note: the test conditions were HCl, pH 1.2 (2 h) + phosphate buffer, pH 6.8 (22 h). | | | | | | | | |

As can be seen from Table 12 and FIG. 6, both formulations 1 and 2 achieved the sustained-release effect. Compared with formulation 1, formulation 2 exhibited reduced and slowed release of saccharides, The releasing amount of saccharide was less than 30% at 8 h, and was significantly increased at 24 h but still less than 70%.

### Example 8: Coated tablet of sustained-release glucose composition

A coated sustained-release glucose tablet was prepared according to the procedures in Example 3.

The composition of the core part is formulation 1 in Example 7, and the coating part is shown in Table 13 below. By weight of the core part, the percentage of the coating was 20% ± 1%.

**Table 13: Formulation of coating part of sustained-release glucose composition**

| Component | Material | Formulation 1 | | Formulation 2 | | Formulation 3 | |
|---|---|---|---|---|---|---|---|
| | | Amount (mg) | Proportion (%) | Amount (mg) | Proportion (%) | Amount (mg) | Proportion (%) |
| Film-forming agent | Ethylcellulose | - | - | 238.1 | 80.33 | - | - |
| | Hydroxypropyl methylcellulose (viscosity: 5 mPa·s) | - | - | - | - | 238.1 | 80.33 |
| Plasticizer | Triethyl citrate | - | - | 26.2 | 8.84 | 26.2 | 8.84 |
| Anti-adherent | Glycerol monostearate | | | 8.3 | 2.80 | 8.3 | 2.80 |
| | Magnesium stearate | | | 23.8 | 8.03 | 23.8 | 8.03 |
| Solvent | Ethanol | - | - | Sufficient | Sufficient | Sufficient | Sufficient |

| | | | | | | | |
|---|---|---|---|---|---|---|---|
| Note: all the materials were of pharmaceutical grade. | | | | | | | |

The coated sustained-release glucose compositions of the 3 formulations were tested for dissolution according to "method 1 (the basket method)" recorded in the General Chapter 0931 - Dissolution and Drug Release Test in Chinese Pharmacopoeia (2020 Edition, Volume IV) using the peroxidase-glucose oxidase (PGO) technique. The specific test conditions are detailed in Example 6. For formulations 1-3 shown in Table 12, the dissolution medium was a HCl solution at pH 1.2 for the first 2 h and a potassium dihydrogen phosphate-sodium hydroxide buffer at pH 6.8 for the last 22 h.

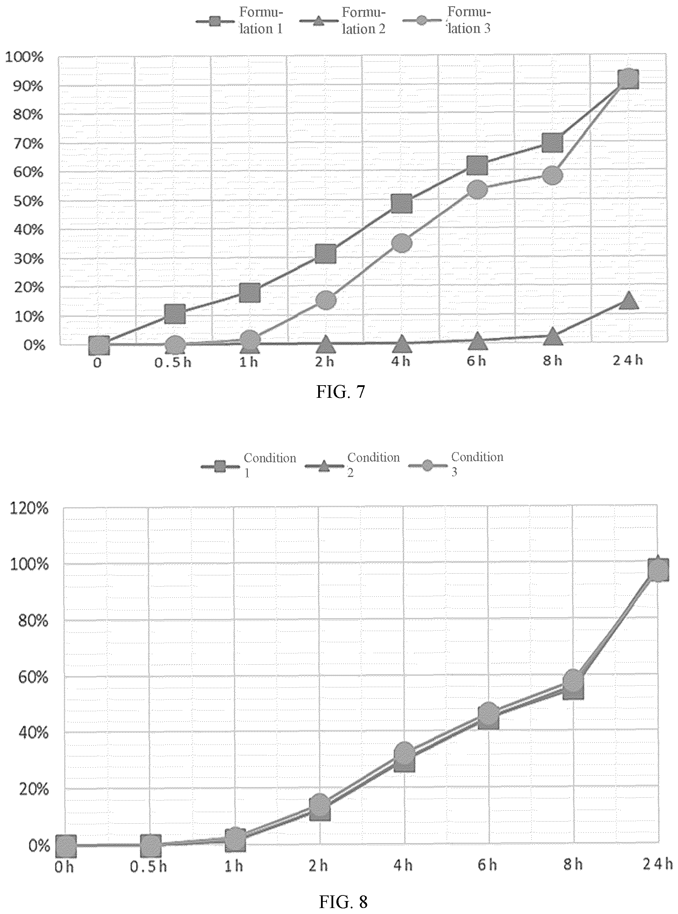

By the above method, the percentage of dissolved glucose by weight was calculated (Table 14) and the time curve was plotted (FIG. 7).

**Table 14: Glucose dissolution results for sustained-release glucose compositions with different coatings**

| Formulatio n | Percentage of dissolved glucose by weight (%) | | | | | | | |
|---|---|---|---|---|---|---|---|---|
| | 0 | 0.5h | 1h | 2h | 4h | 6h | 8h | 24h |
| 1 | 0 | 10.6 | 17.8 | 31.2 | 48.4 | 61.7 | 69.4 | 91.1 |
| 2 | 0 | 0 | 0 | 0 | 0 | 0.90 | 2.30 | 14.50 |
| 3 | 0 | 0 | 1.6 | 15.0 | 34.8 | 53.4 | 58.1 | 91.9 |

| | | | | | | | | |
|---|---|---|---|---|---|---|---|---|
| Note: the test conditions were HCl, pH 1.2 (2 h) + phosphate buffer, pH 6.8 (22 h). | | | | | | | | |

As can be seen from Table 14 and FIG. 7, all formulations achieved the sustained release of the saccharide. Compared with formulation 1, formulation 2 exhibited significantly reduced and slowed release of glucose with a releasing amount of less than 15% at 24 h. Formulation 3 exhibited a reduced releasing amount of glucose in the first two hours of less than or equal to 15%; the releasing amount gradually increased over the next 6 h, and was less than 35% at 4 h but greater than 55% at 8 h; the releasing amount was greater than 90% at 24 h.

### Example 9: Preparation of sustained-release glucose composition

The amounts of materials of the tablet core part are shown in formulation 2 in Example 6, and the formulation of the coating part is shown in formulation 3 in Example 7. The preparation procedures are detailed as follows:
(1) Core part
   a. Pretreatment: A proper amount of hydroxypropyl methylcellulose (viscosity: 5 mPa·s) according to the formulation was slowly added to and uniformly dispersed in hot water at vortex to avoid agglomeration; the dispersion was cooled, stirred for about 1 h, and cooled to room temperature to give a solution.
   b. Granulation: Proper amounts of glucose and microcrystalline cellulose according to the formulation were sieved through a screen; after sieving, the materials were loaded into a quick mixing granulator for mixing to give a uniform dry mixture; the dry mixture was granulated in a mixing granulator with a hydroxypropyl methylcellulose binder for 1-3 min; the granulated product was fed into a mill equipped with a screen to give a wet granule.
   c. Drying: The wet granule was loaded onto a fluidized bed and dried at an inlet temperature of 60 °C ± 5 °C with a weight loss of less than 5%; the product was fed into a mill equipped with a screen to give a uniform granule.
   d. Blending: Proper amounts of hydroxypropyl methylcellulose (viscosity: 100000 mPa·s) and sodium alginate according to the formulation were sieved through a screen mesh; after sieving, the materials were mixed in a blender for 5 minutes; a proper amount of magnesium stearate according to the formulation was sieved and added into the blender for lubrication, and the mixture was blended for 5 min.
   e. Tableting: The compositions were tableted in a high-speed tablet press using a standard female punch (20 mm × 10 mm), with the average tablet weight controlled at about 1400 mg and the hardness controlled at 80-280 N.
(2) Coating part
   a. Preparation of coating solution: Proper amounts of hydroxypropyl methylcellulose (viscosity: 5 mPa·s) and triethyl citrate according to the formulation were added into an ethanol solution before water was slowly added, and the mixture was uniformly mixed; proper amounts of magnesium stearate and glycerol monostearate according to the formulation were added into another ethanol solution, and the mixture was homogenized; finally, the two solutions were mixed to give the coating solution.
   b. Coating: tablet cores were loaded onto a coating machine and pre-heated at 40 ± 1 °C; then the coating solution was sprayed on the pre-heated tablet cores to give a uniform coating until a weight gain of 19%-21%; finally, the coated tablets were dried at 40 ± 1 °C for 120 min.

### Example 10: Dissolution exploration of sustained-release glucose composition

The dissolution of the sustained-release glucose composition was examined by simulating different gastrointestinal pH environments.

The prepared sustained-release glucose composition was tested for dissolution according to "method 1 (the basket method)" recorded in the General Chapter 0931 - Dissolution and Drug Release Test in Chinese Pharmacopoeia (2020 Edition, Volume IV) using the peroxidase-glucose oxidase (PGO) technique. The specific test conditions are detailed in Example 6. For condition 1 in Table 15, the dissolution medium was a HCl solution at pH 1.2 for the first 2 h and a sodium acetate trihydrate-glacial acetic acid buffer at pH 5.5 for the last 22 h; for condition 2 in Table 15, the dissolution medium was a HCl solution at pH 1.2 for the first 2 h and a potassium dihydrogen phosphate-sodium hydroxide buffer at pH 6.8 for the last 22 h; for condition 3 in Table 15, the dissolution medium was a HCl solution at pH 1.2 for the first 2 h and a potassium dihydrogen phosphate-sodium hydroxide buffer at pH 7.4 for the last 22 h.

The sustained-release glucose composition was tested for dissolution in media at three different pH gradients simulating the pH of 3 gastric (acidic) and intestinal (basic) circumstances. The dissolution amount of the sustained-release glucose composition over 8 hours was measured using the method described above (Table 15), and the glucose releasing profiles were plotted (as shown in FIG. 8).

**Table 15: Glucose dissolution results for sustained-release glucose composition in different pH conditions**

| Time (h) | Releasing proportion (%) | | |
|---|---|---|---|
| | Condition 1 HCl, pH 1.2 (2 h) + acetic acid buffer, pH 5.5 (6 h) | Condition 2 HCl, pH 1.2 (2 h) + phosphate buffer, pH 6.8 (6 h) | Condition 3 HCl, pH 1.2 (2 h) + phosphate buffer, pH 7.4 (6 h) |
| 0 | 0.0 | 0.0 | 0.0 |
| 0.5 | 0.0 | 0.1 | 0.0 |
| 1 | 1.5 | 1.5 | 2.4 |
| 2 | 12.3 | 12.2 | 14.0 |
| 4 | 29.7 | 30.2 | 32.4 |
| 6 | 44.7 | 44.4 | 46.3 |
| 8 | 54.9 | 56.0 | 57.7 |
| 24 | 97.0 | 98.2 | 96.5 |

As can be seen from Table 15 and FIG. 8, in all pH conditions, the sustained-release glucose composition exhibited, on the basis of the original mass, a 2-h releasing amount less than 15%, a 4-h releasing amount less than 35%, an 8-h releasing amount greater than 50%, and a 24-h releasing amount greater than 90%. The above results suggest that on one hand, the sustained-release glucose composition is not susceptible to changes in pH condition and is adaptive to the gastrointestinal tract environment with acidity changes; on the other hand, the releasing profile of glucose in the sustained-release glucose composition matches with the characteristic nocturnal hypoglycemia in diabetes patients, such that the use of the composition before sleep can effectively control the blood glucose, so as to prevent and control nocturnal hypoglycemia.

### Example 11: Stability test of sustained-release glucose composition

The stability of the sustained-release glucose composition prepared in Example 9 was investigated.

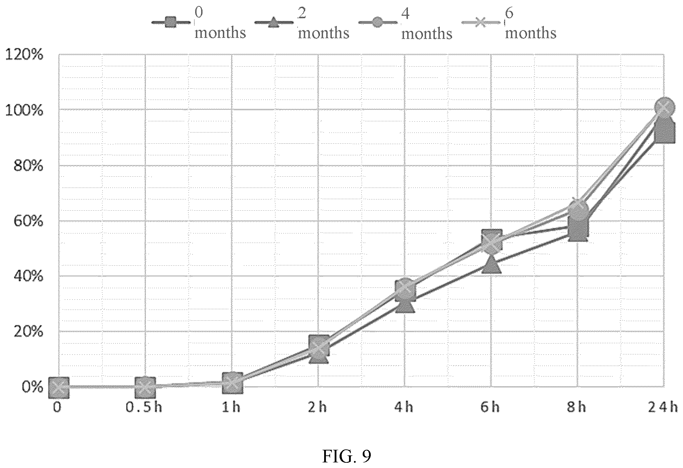

The prepared sustained-release glucose composition was tested for dissolution according to "method 1 (the basket method)" recorded in the General Chapter 0931 - Dissolution and Drug Release Test in Chinese Pharmacopoeia (2020 Edition, Volume IV) using the peroxidase-glucose oxidase (PGO) technique. The specific test conditions are detailed in Example 6. At the four time points shown in Table 16, the dissolution medium was a HCl solution at pH 1.2 for the first 2 h and a potassium dihydrogen phosphate-sodium hydroxide buffer at pH 6.8 for the last 22 h. The releasing data of glucose from the sustained-release glucose composition at baseline and after two, four, and six months (as shown in Table 16) were obtained, and the glucose releasing profiles were plotted (as shown in FIG. 9).

**Table 16: Glucose dissolution results for sustained-release glucose composition at different time**

| Time | Percentage of dissolved glucose by weight (%) | | | | | | | |
|---|---|---|---|---|---|---|---|---|
| | 0 | 0.5h | 1h | 2h | 4h | 6h | 8h | 24h |
| 0 | 0 | 0 | 1.6 | 15.0 | 34.8 | 53.4 | 58.1 | 91.9 |
| 2 months | 0 | 0.1 | 1.5 | 12.2 | 30.2 | 44.4 | 56.0 | 98.2 |
| 4 months | 0 | 0.1 | 1.8 | 14.2 | 35.8 | 51.5 | 64.1 | 101.1 |
| 6 months | 0 | 0 | 1.5 | 14.1 | 36.2 | 51.7 | 66.5 | 100.7 |

| | | | | | | | | |
|---|---|---|---|---|---|---|---|---|
| Note: the test conditions were HCl, pH 1.2 (2 h) + phosphate buffer, pH 6.8 (22 h). | | | | | | | | |

As can be seen from Table 16 and FIG. 9, after six months of preservation, the dissolution of glucose was substantially identical to that at baseline, i.e., on the basis of the original mass, a 2-h releasing amount less than or equal to 15%, a 4-h releasing amount less than 40%, an 8-h releasing amount greater than 55%, and a 24-h releasing amount greater than 90%. The results demonstrated that the sustained-release glucose composition prepared according to Example 9 has good stability.

While specific examples of the present invention have been described, it will be apparent to those skilled in the art that various changes and modifications can be made to the present disclosure without departing from the spirit and scope of the present invention. Therefore, the appended claims encompass all such changes and modifications that fall within the scope of the present disclosure.

## Claims

1. A sustained-release saccharide composition, comprising a saccharide and a sustained-release agent, wherein the sustained-release agent comprises at least two selected from hydroxypropyl methylcellulose, sodium alginate, cellulose, methylcellulose, ethylcellulose, microcrystalline cellulose, carboxymethylcellulose, sodium carboxymethylcellulose, hydroxyethyl cellulose, hydroxypropyl cellulose, polyethylene oxide, carboxyvinyl polymer, polyoxyethylene, aqueous polyvinyl acetate dispersion, glycerol monooleate, glycerol monostearate, carbomer homopolymer, carbomer interpolymer, carrageenan, shellac, guar gum, xanthan gum, alginic acid, and starch.

2. The sustained-release saccharide composition according to claim 1, further comprising a diluent and a glidant; preferably, further comprising a diluent, a glidant, and a binder.

3. The sustained-release saccharide composition according to claim 1, wherein the saccharide is selected from glucose, maltose, lactose, galactose, fructose, mannose, xylose, sucrose, fucose, arabinose, rhamnose, ribose, sorbose, lactulose, tagatose, maltulose, palatinose, isomaltose, trehalose, sophorose, rutinose, kojibiose, nigerose, laminaribiose, gentiobiose, turanose, gentiobiulose, mannobiose, melibiulose, xylobiose, sorbitol, lactitol, mannitol, erythritol, and a combination thereof;
preferably, the saccharide is present in a percentage of 45%-85%, preferably 60%-80%, by weight of the sustained-release composition;
more preferably, the saccharide is glucose or maltose, and is present in a percentage of 50%-80%, preferably 70%-80%, by weight of the sustained-release composition.

4. The sustained-release saccharide composition according to claim 1, wherein the sustained-release agent is present in a percentage of 5%-40%, preferably 12%-24%, by weight of the sustained-release composition;
more preferably, the sustained-release agent is a combination of hydroxypropyl methylcellulose and sodium alginate, and is present in a percentage of 5%-35%, preferably 10%-30%, and more preferably 15%-25%, by weight of the sustained-release composition;
preferably, the viscosity of the hydroxypropyl methylcellulose is 75000-140000 mPa·s, preferably 90000-120000 mPa·s, and more preferably 100000 mPa·s;
preferably, the viscosity of the hydroxypropyl methylcellulose is 11250-21000 mPa·s, preferably 13000-18000 mPa·s, and more preferably 15000 mPa·s.

5. The sustained-release saccharide composition according to claim 1, wherein the sustained-release saccharide composition is in the form of a granule or a tablet; preferably the granule or the tablet further comprises a coating part; more preferably, the coating part comprises a film-forming agent, a plasticizer, and an anti-adherent; preferably, the film-forming agent of the coating part is selected from hydroxypropyl methylcellulose, hydroxypropyl cellulose, sodium methylcellulose, and an acrylate polymer;
preferably, the film-forming agent is present in a percentage of 55%-95%, preferably 65%-85%, by weight of the coating part;
more preferably, the film-forming agent is hydroxypropyl methylcellulose, and is present in a percentage of 65%-95%, preferably 75%-85%, by weight of the coating part;
wherein, the viscosity of the hydroxypropyl methylcellulose is 1-20 mPa·s, preferably 2-15 mPa·s, more preferably 3-10 mPa·s, and most preferably 5 mPa·s.

6. The sustained-release saccharide composition according to claim 5, wherein the plasticizer of the coating part is selected from castor oil, coconut oil, glycerin, propylene glycol, low-molecular-weight polypropylene glycol, polysorbate, a sorbitan ester, triethyl citrate, diethyl phthalate, dibutyl phthalate, tributyl citrate, and a combination thereof;
preferably, the plasticizer is present in a percentage of 5%-20%, preferably 5%-15%, by weight of the coating part;
more preferably, the plasticizer is triethyl citrate, and is present in a percentage of 5%-18%, preferably 5%-10%, by weight of the coating part;
preferably, the anti-adherent in the coating part is selected from magnesium stearate, magnesium silicate, stearic acid, glycerol stearate, glycerol monostearate, sodium stearate, talc, colloidal silica, magnesium oxide, magnesium trisilicate, and a combination thereof;
preferably, the anti-adherent is present in a percentage of 2%-20%, preferably 5%-15%, by weight of the coating part;
more preferably, the anti-adherent is a combination of glycerol monostearate and magnesium stearate, and is present in a percentage of 2%-15%, preferably 2%-12%, by weight of the coating part;
preferably, the coating part of the sustained-release saccharide composition comprises, by weight of the coating part: about 80.33% of hydroxypropyl methylcellulose, about 8.83% of triethyl citrate, about 2.81% of glycerol monostearate, and about 8.03% of magnesium stearate, wherein the viscosity of the hydroxypropyl methylcellulose is 5 mPa·s;
preferably, a core part of the sustained-release saccharide composition comprises, by weight of the core part: about 79.24% of maltose, about 16.64% of hydroxypropyl methylcellulose, about 1.11% of sodium alginate, about 1.9% of microcrystalline cellulose, and about 1.11% of magnesium stearate, wherein the viscosity of the hydroxypropyl methylcellulose is 15000 mPa·s.

7. The sustained-release saccharide composition according to claim 5, wherein the coating part is present in a percentage of 2%-8%, preferably 3%-6%, by weight of a core part.

8. The sustained-release saccharide composition according to claim 5, wherein a core part of the sustained-release saccharide composition comprises, by weight of the core part: about 71.43% of glucose, about 24% of hydroxypropyl methylcellulose, about 1% of sodium alginate, about 1.71% of microcrystalline cellulose, about 1% of magnesium stearate, and about 0.86% of hydroxypropyl methylcellulose, wherein the viscosity of the hydroxypropyl methylcellulose of about 24% is 100000 mPa·s and the viscosity of the hydroxypropyl methylcellulose of about 0.86% is 15 mPa·s; preferably, the coating part is present in a percentage of 15%-25%, preferably 18%-22%, by weight of the core part.

9. A method for preparing the sustained-release saccharide composition according to claim 1, comprising:
mixing the saccharide and the sustained-release agent of the sustained-release saccharide composition according to claim 1 by direct granulation, dry granulation, or wet granulation to give a granule.
